# EUROPÄISCHE PATENTSCHRIFT

(11) **EP 3 270 803 B1**
(45) Veröffentlichungstag und Bekanntmachung des Hinweises auf die Patenterteilung: **23.10.2019**
(21) Anmeldenummer: 16710174.0
(22) Anmeldetag: 16.03.2016
(51) Int. Cl.: A61B 17/70

(54) **POLYAXIALE PEDIKELSCHRAUBE MIT KUGELSEGMENTFÖRMIGEM KOPF**
POLYAXIAL PEDICLE SCREW WITH A HEAD IN THE SHAPE OF A BALL SEGMENT
VIS PÉDICULAIRE POLYAXIALE MUNIE D'UNE TÊTE SEMI-SPHÉRIQUE

(30) Priorität: 19.03.2015 DE 102015003574; 17.08.2015 DE 102015010741
(43) Veröffentlichungstag der Anmeldung: 24.01.2018
(73) Patentinhaber: NGMedical GmbH, 66620 Nonnweiler-Primstal (DE)
(72) Erfinder: BACKES, Hermann, 66640 Namborn (DE)
(74) Vertreter: Kruspig, Volkmar
(86) Internationale Anmeldenummer: PCT/EP2016/055664
(87) Internationale Veröffentlichungsnummer: WO 2016/146672

(56) Entgegenhaltungen:
- WO-A1-2005/023125
- DE-U1- 9 402 695
- US-A1- 2008 167 688
- US-A1- 2009 312 804
- US-A1- 2012 083 845

## Beschreibung

Die Erfindung betrifft eine polyaxiale Pedikelschraube mit kugelsegmentförmigem Kopf und Gewindeschraubenschaft, wobei im Kopf ein Hohlkugelsegment mit Aussparung zur Aufnahme eines stabförmigen Verbinders gelagert ist, um Schwenk- und Drehbewegungen des Verbinders zu ermöglichen, wobei weiterhin in Längsachsenrichtung des Gewindeschraubenschafts der kugelsegmentförmige Kopf eine Aussparung mit Innengewinde aufweist, in welche der stabförmige Verbinder hinein- und herausbewegbar ist und mittels des Innengewindes in der Aussparung eine Madenschraube zum Blockieren des Hohlkugelsegments einbringbar ist, gemäß Oberbegriff des Anspruchs 1.

Aus der DE 10 2012 202 750 A1 ist eine dynamische Stabilisierungseinrichtung für Knochen, insbesondere für die Wirbelsäule vorbekannt. Das Stabilisierungssystem umfasst zwei Aufnahmeelemente nebst einem federelastischen Element. Die Aufnahmeelemente sind verschiebbar zueinander gelagert und erlauben Bewegungen in Zug- und Druckrichtung des Implantats. Die Aufnahmeelemente sind mit Hilfe von Auflageflächen und hakenähnlichen Gegenflächen so ausgestaltet, dass das federelastische Element zwischen den Aufnahmeelementen ausschließlich einer Druckbeanspruchung ausgesetzt wird, selbst dann, wenn das Verbindungselement als Ganzes auf Zug beansprucht wird. Das Federelement kann aus einer Spiralfeder, einer Tellerfeder oder einem elastischen Polymer mit massivem oder hohlem Querschnitt bestehen. Enden der Aufnahmeelemente werden in U-förmigen Ausnehmungen eines Schraubenkopfes einer Pedikelschraube fixiert, wobei die Pedikelschrauben einen Gewindeschraubenschaft umfassen. Die stabförmigen Enden der Aufnahmeelemente sind bezogen auf die Position im Schraubenkopf fix und unbeweglich.

Bei der dynamischen Stabilisierungseinrichtung für Knochen nach DE 102 36 691 B4 ist wenigstens ein erstes und ein zweites Knochenverankerungselement vorgesehen mit jeweils einem ersten, in einem Knochen zu verankernden Abschnitt und einem zweiten, mit einem Stab zu verbindenden Abschnitt. Die Knochenverankerungselemente werden über einen Stab verbunden, wobei diese Verbindung in Richtung der Stabachse verschiebbar und zumindest eines der Knochenverankerungselemente als Polyaxial-Knochenschraube vorgesehen ist. Zwischen den Knochenverankerungselementen kann ein in Richtung der Stabachse elastisch vorspannbares Element vorgesehen sein. Bei den eingesetzten Polyaxial-Knochenschrauben handelt es sich um solche, die schraubenkopfseitig die Form eines Kugelsegments besitzen, wobei das entsprechende Kugelsegment von einer Hülse umfasst ist, welche wiederum der Befestigung des Stabes dient. Die Stabfixierung erfolgt durch Einschrauben einer Innenschraube. Die Innenschraube umfasst bei einer Ausführungsform einen Gleitboden, um ein reibungsarmes Bewegen des Stabes zu ermöglichen.

Bei der gattungsbilden DE 10 2005 005 647 A1 wird von einer Vorrichtung zur Stabilisierung der Wirbelsäule, umfassend mindestens zwei Pedikelschrauben und mindestens ein dazwischenliegendes Verbindungselement ausgegangen. Die dortigen Pedikelschrauben verbinden die Vorteile einer Monoaxialschraube mit denen einer Polyaxialschraube, indem der Schraubenkopf auf dem Schraubenschaft unbeweglich gelagert wird, um eine optimale Reponierbarkeit der Wirbel zu gewährleisten und sich in dem Schraubenkopf ein bewegliches Kugelelement befindet, welches die Einführung des durch die Schraubenköpfe verlaufenden Verbindungselements erleichtert und nach erfolgter Einführung fest fixieren kann.

Insofern umfasst die in der DE 10 2005 005 647 A1 näher beschriebene Pedikelschraube einen Schraubenschaft und einen Schraubenkopf mit zwei gegenüberliegenden langlochförmigen Aussparungen. Im Schraubenkopf ist ein Kugelelement mit einer Aussparung zur Aufnahme eines stabförmigen Verbindungselements beweglich gelagert. Hierdurch wird erreicht, dass sich das Kugelelement in Anteflexions- und Retroflexions-Richtung der Lage des durchgeführten Verbindungselements anpassen und dieses nach erfolgter Fixierung einschließen kann. Der Schraubenkopf selbst ist hohl, um das Kugelelement aufzunehmen, das Drehbewegungen eines hindurchgeführten Verbindungselements im Schraubenkopf um eine laterale Achse ermöglicht. Um nun das durchlaufende Verbindungselement diese Bewegungen ausführen lassen zu können, weist der Schraubenkopf zwei langlochförmige gegenüberliegende Öffnungen auf, innerhalb derer die Kippbewegungen oder Neigebewegungen des Verbindungselements realisierbar sind. Hieraus erfolgt eine bevorzugte zylindrische oder ovale Ausgestaltung des Schraubenkopfes. Als Material für die vorbekannten Pedikelschrauben wird medizinischer Edelstahl, Titan oder Titanlegierungen, Tantal, aber auch Kunststoff eingesetzt.

Die vorstehend zitierten Lösungen zeigen also bewegliche Kugelelemente zur Winkelvariabilität auf, wobei jedoch elastische Eigenschaften nicht gegeben sind. Lösungen mit Federelementen, die zwischen den Verbindungsstäben eines Implantats ausgebildet sind, gewährleisten zwar eine entsprechende Elastizität zum Zweck einer dynamischen Stabilisierung entsprechender Wirbelkörper, jedoch nehmen derartige zwischen den Stäben vorhandene Federelemente ein nicht zu vernachlässigendes Volumen ein, was es vom Grundsatz her zu vermeiden gilt.

Aus dem Vorgenannten ist es daher Aufgabe der Erfindung, eine weiterentwickelte polyaxiale Pedikelschraube mit kugelsegmentförmigem Kopf und Gewindeschraubenschaft anzugeben, wobei im implantierten Zustand eine physiologische Bewegung durch mögliche Längenänderung und gleichzeitige Verkippung stabförmiger Verbinder in Relation zur fixen Pedikelschraube möglich sein soll. Auf diese Weise kann die Anordnung aus Pedikelschrauben und Verbinder die Bewegungen der Wirbelsäule wie Flexion, Extension, Seitenneigung und Rotation, aufnehmen und dieser Bewegung folgen.

Die Lösung der Aufgabe der Erfindung erfolgt durch die Merkmalskombination gemäß der Lehre des Patentanspruchs 1, wobei die Unteransprüche mindestens zweckmäßige Ausgestaltungen und Weiterbildungen umfassen.

Ein erfindungsgemäßer Ansatz bei der Ausbildung der speziellen polyaxialen Pedikelschraube mit kugelsegmentförmigem Kopf besteht darin, alle notwendigen Funktionen einschließlich Aufnahme und Führung stabförmiger Verbinder nebst den gewünschten Dämpfungseigenschaften mit gezielter Beweglichkeit im Schraubenkopf selbst zu konzentrieren, so dass ansonsten zwischen den Verbindern eingesetzte elastische Mittel in Fortfall kommen können.

Die Erfindung löst das Problem bekannter Systeme, das darin besteht, dass derartige Konstruktionen so viel Raum einnehmen, dass bei eng aneinander stehenden Pedikeln, wie z.B. dem untersten Segment L5/S1, eine Implantation nahezu unmöglich ist.

Es wird demnach von einer polyaxialen Pedikelschraube mit kugelsegmentförmigem Kopf und Gewindeschraubenschaft ausgegangen, wobei im Kopf ein Hohlkugelsegment mit Ausnehmungen zur Aufnahme des stabförmigen Verbinders gelagert ist. Das Hohlkugelsegment ermöglicht Schwenk- und Drehbewegungen des Verbinders. In Längsachsenrichtung des Gewindeschraubenschafts besitzt der kugelsegmentförmige Kopf eine Aussparung mit Innengewinde, in welche der stabförmige Verbinder hinein- und herausschwenkbar ist. Mittels einer Madenschraube, die in das Innengewinde der Aussparung eingebracht werden kann, ist ein Blockieren des Hohlkugelsegments möglich.

Erfindungsgemäß ist im Hohlkugelsegment ein elastischer Kunststoff-Pufferkörper eingesetzt, welcher mit einem Stößel in Verbindung steht, wobei der Stößel einen Kopf aufweist, welcher in eine diesbezügliche Ringnut des Kunststoff-Pufferkörpers eingreift.

In einer bevorzugten Ausführungsform ist der Kopf des Stößels vom Kunststoff-Pufferkörper umspritzt und diesbezüglich mit diesem formschlüssig verbunden.

Weiterhin besitzt der Kunststoff-Pufferkörper einen umlaufenden Führungsring und einen endseitigen, sphärischen Führungsanschlag, wobei dem Führungsanschlag gegenüberliegend eine Öffnung für den Stößel ausgebildet ist. Der Rand der Öffnung stützt sich an einer Schale ab, welche ihrerseits an der Innenseite des Hohlkugelsegments anliegt, derart, dass der Kunststoff-Pufferkörper mit seinem Führungsanschlag im kugelsegmentförmigen Kopf unter Vorspannung fixiert ist.

Der Stößel weist einen Abschnitt, z.B. einen Gewindeabschnitt zum Befestigen des erwähnten stabförmigen Verbinders auf.

Im Führungsring und/oder im Führungsanschlag sind Nuten oder Aussparungen zum Flüssigkeitsdurchtritt ausgebildet.

Über das Design des Kunststoff-Pufferkörpers hinsichtlich Geometrie und/oder Material sind Radial-, Zug- und Druckkräfte und diesbezüglich erreichbare Hub- und Schwenkbewegungen des Stößels und des daran fixierten Verbinders vorgebbar.

Bei einer Ausführungsform der Erfindung besteht der Kunststoff-Pufferkörper aus Polycarbonaturethan (PCU) oder ähnlichen Kunststoffen.

Das Hohlkugelsegment ist mittels eines Klemmrings im kugelsegmentförmigen Kopf gesichert.

Bei einer Ausgestaltung der Erfindung besteht das Hohlkugelsegment aus Titanmaterial oder einer Titanlegierung. Die Schale selbst kann ebenfalls aus einer Titanlegierung, aber auch aus einem Keramikmaterial gefertigt sein.

Der Gewindeschraubenschaft der Pedikelschraube weist bevorzugt ein selbstschneidendes Gewinde mit geringer Steigung für eine optimale Einschraubposition auf.

Bei einer Ausgestaltung der Erfindung weist der Kunststoff-Pufferkörper im Bereich des Führungsanschlags einen Zugang zum Kopf des Stößels auf. Im Kopf des Stößels kann ein Rücksprung mit einer Kontur zur Aufnahme eines Werkzeugs eingebracht sein, um bei der Montage des Verbinders am Stößel ein Gegenhalten zu ermöglichen.

Bei einer erfindungsgemäßen Weiterbildung ist der kugelsegmentförmige Kopf geteilt ausgebildet. Die Teilung erfolgt bevorzugt derart, dass zwei Halbkugeln bzw. Halbkugelsegmente entstehen, die entlang ihrer Trennungslinie über Mittel verfügen, die es gestatten, eine formschlüssige Verbindung der Halbschalen zu bewirken. Dies können beispielsweise Rastmittel und Rastgegenmittel sein. Die Verbindung erfolgt bevorzugt unter Nutzung des Prinzips eines Bajonettverschlusses.

Die Erfindung soll nachstehend anhand eines Ausführungsbeispiels sowie unter Zuhilfenahme von Figuren näher erläutert werden.

Hierbei zeigen:

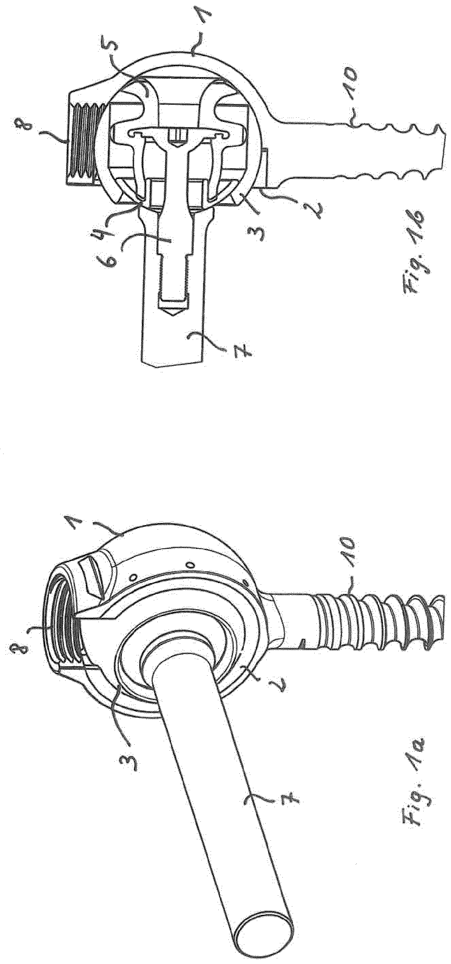

- Fig. 1a und 1b: eine perspektivische Ansicht sowie eine Schnittdarstellung der erfindungsgemäßen polyaxialen Pedikelschraube mit angedeutetem Verbinder, Stößel und Kunststoff-Pufferkörper;

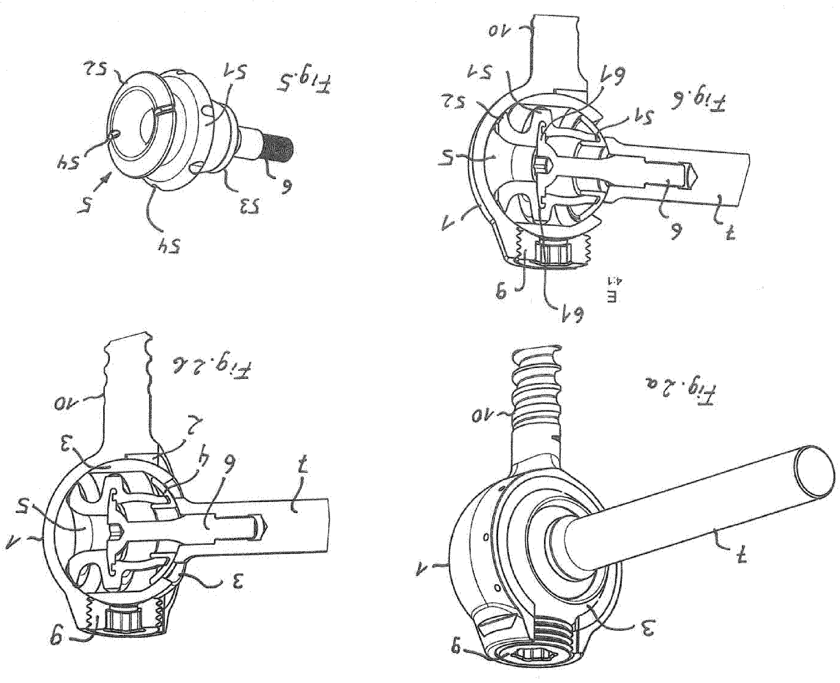

- Fig. 2a und 2b: eine perspektivische Darstellung und eine Schnittdarstellung ähnlich derjenigen nach Fig. 1a und 1b, jedoch mit eingebrachter Madenschraube in der Aussparung mit Innengewinde im kugelsegmentförmigen Kopf;

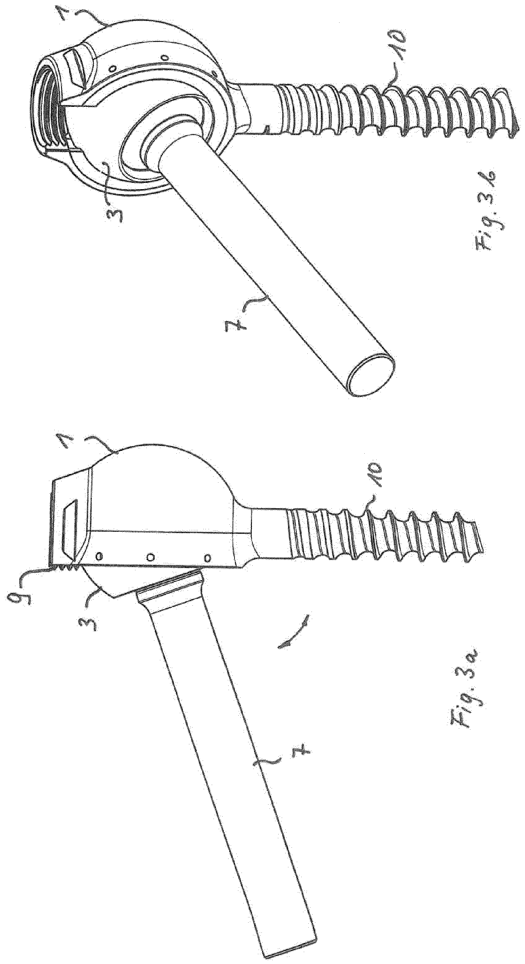

- Fig. 3a und 3b: verschiedene perspektivische Ansichten mit einer 20° Polyaxialverschwenkung zwischen der Längsachse des Gewindeschraubenschafts und dem Verbinder;

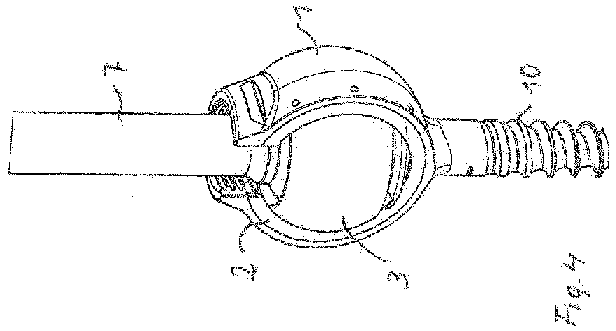

- Fig. 4: eine Darstellung der Anordnung des Verbinders, eingeschwenkt in die Aussparung zum Zweck des ungehinderten Einschraubens des Implantats;
- Fig. 5: eine perspektivische Darstellung des Kunststoff-Pufferkörpers mit Stößel;
- Fig. 6: eine Detaildarstellung der Pedikelschraube in Schnittansicht;
- Fig. 7: eine Darstellung der erfindungsgemäßen Pedikelschraube mit kugelsegmentförmigem Kopf, welcher in zwei Halbschalen bzw. Halbschalensegmente geteilt ist, wobei die Segmente oder Halbschalen über Rastmittel und Rastgegenmittel verbindbar gestaltet sind, in einer getrennten Position und
- Fig. 8: eine Darstellung ähnlich derjenigen nach Fig. 7, jedoch mit verbundener Position der Halbschalen.

Die erfindungsgemäße polyaxiale Pedikelschraube, wie in den Figuren dargestellt, umfasst einen kugelsegmentförmigen Kopf 1, der in seinem Fußbereich in einen Gewindeschraubenschaft 10 übergeht.

Im kugelsegmentförmigen Kopf 1 ist ein Hohlkugelsegment 3 eingesetzt, welches eine Ausnehmung zur Aufnahme eines stabförmigen Verbinders 7 umfasst. Das Hohlkugelsegment 3 ist innerhalb des kugelsegmentförmigen Kopfes 1 verschwenkbeweglich. Diesbezüglich können entsprechende Schwenk- und Drehbewegungen des Verbinders 7 erfolgen.

In Längsachsenrichtung des Gewindeschraubenschafts 10 weist der kugelsegmentförmige Kopf 1 eine Aussparung 8 mit Innengewinde auf.

Während des Einschraubens der Pedikelschraube wird der Verbinder mitsamt Hohlkugelsegment in eine solche Position verbracht, dass der Verbinder in der Aussparung 8 zu liegen kommt.

Nach Einschrauben der Pedikelschraube in das Knochen-, insbesondere Wirbelkörpermaterial kann der Verbinder in die dann erforderliche Lage positioniert werden, wobei über ein Innengewinde in der Aussparung 8 eine Madenschraube 9 ein Blockieren des Hohlkugelsegments 3 gestattet, wodurch auch der Verbinder 7 lagefixiert wird. Auch nach dem Fixieren des Hohlkugelsegmentes 3 bleibt der Stab gegenüber der Hohlkugel schwenkbeweglich.

Im Hohlkugelsegment 3 befindet sich ein Kunststoff-Pufferkörper 5, welcher mit einem Stößel 6 in Verbindung steht, wobei der Stößel 6 einen Kopf 61 aufweist, welcher in eine diesbezügliche Ringnut im Kunststoff-Pufferkörper eingreift bzw. vom Kunststoff-Pufferkörper im Kopfbereich 61 umspritzt ist.

Der Kunststoff-Pufferkörper 5 besitzt einen umlaufenden Führungsring 51 und einen endseitigen, sphärischen Führungsanschlag 52. Dem Führungsanschlag 52 im Wesentlichen gegenüberliegend ist im Kunststoff-Pufferkörper 5 eine Öffnung für den Stößel 6 ausgebildet.

Der Rand 53 der Öffnung des Kunststoff-Pufferkörpers 5 stützt sich an einer Schale 4 ab, welche ihrerseits an der Innenseite des Hohlkugelsegments 3 anliegt, und zwar derart, dass der Kunststoff-Pufferkörper 5 mit seinem Führungsanschlag 52 im kugelsegmentförmigen Kopf unter Vorspannung fixiert ist. Durch die elastischen Eigenschaften des Kunststoff-Pufferkörpers 5 bleibt eine Beweglichkeit des Stößels und damit des Verbinders 7 erhalten, so dass physiologische Bewegungen durch eine mögliche Längenänderung und gleichzeitige Verkippung des Verbinders in Relation zur fixen Schraube erfolgen können. Hierdurch kann die Anordnung die vorhandenen Bewegungen in der Wirbelsäule wie Flexion, Extension, Seitenneigung und Rotation vollziehen.

Durch die erfindungsgemäße Konstruktion wird der Kunststoff-Pufferkörper 5 nur auf Druck belastet, so dass ein möglicher Verschleiß reduziert ist.

Im Führungsring 51 und/oder im Führungsanschlag 52 sind Nuten oder Aussparungen 54 zum Flüssigkeitsdurchtritt ausgebildet. Eingetretene Flüssigkeiten können also mit Blick auf die Aussparung 54 überströmen. Darüber hinaus kann die Wirkung des Kunststoff-Pufferkörpers 5 gezielt beeinflusst werden.

Über die Designgestaltung des Kunststoff-Pufferkörpers 5 besteht die Möglichkeit, die Zug- und Druckseite individuell einzustellen und damit maximale Hübe und Kräfte als auch den Kraftverlauf zu variieren. Hierdurch können gezielt Längenänderungen und Bewegungsmöglichkeiten des Verbinders definiert werden. Durch eine Variation der Gestaltung des Kunststoff-Pufferkörpers 5, auch hinsichtlich der Materialauswahl, kann ein zunächst linearer und später progressiver Kräfteverlauf realisiert werden.

Eine gezielte Vorspannung beim Einbringen des Kunststoff-Pufferkörpers im Hohlraum der Kugel greift einem Setzen oder Kriechen des Materials voraus, so dass die notwendige Langzeitstabilität und ein reproduzierbares Verhalten gewährleistet ist.

Das Hohlkugelsegment 3 wird mit Hilfe eines Klemmrings 2 im kugelsegmentförmigen Kopf 1 gesichert.

Im Bereich des Führungsanschlags 52 weist bei einer bevorzugten Ausgestaltung der Kunststoff-Pufferkörper 5 einen Zugang zum Kopf 61 des Stößels 6 auf, um ein Werkzeug in eine dort vorhandene Aussparung einzuführen.

Der kugelförmige Kopf der Pedikelschraube fixiert alle für die erfindungsgemäße Lösung notwendigen Komponenten. Der Klemmring 2 hält diese Komponenten im Inneren des Kopfes der Pedikelschraube. Der Klemmring 2 kann z.B. mittels Passstiften fixiert, aber auch beispielsweise stoffschlüssig, mittels Laserschweißen befestigt werden.

Das Hohlkugelsegment 3 stellt quasi eine Innenhülse dar und bildet die vordere, den offenen Teil des kugelsegmentförmigen Kopfes verschließende Hälfte der Gesamtkugel und klemmt die übrigen Komponenten im Inneren des Kopfes.

Während der Operation beim Einsatz der Pedikelschraube ermöglicht die Innenhülse eine Einstellung des Winkels zwischen Stab und Pedikelschraube in einem Bereich von ca. ± 20° in jede beliebige Richtung, wodurch die gewünschte polyaxiale Funktion gewährleistet ist. Die Fixierung ist im Übrigen im Rahmen dieser Beweglichkeit spannungsfrei. Darüber hinaus bildet die Innenhülse ein Widerlager für die Bewegung der Stützschale 4 und des Kunststoff-Pufferkörpers 5. Wie bereits ausgeführt, wird die Innenhülse 3 mit Hilfe der Madenschraube 9 oder eines ähnlichen Mittels am Ende der Implantation fixiert. Nach dem Fixieren des Hohlkugelsegmentes 3 durch die erwähnte Madenschraube 9 bleibt der Kunststoff-Pufferkörper mit angeschlossenem Verbinder 7 und Schale 4 innerhalb des Hohlkugelsegmentes 3 schwenkbeweglich und gleicht so noch vorhandene Ausrichtungsfehler aus.

Die Schale 4 verschließt die verbleibende Öffnung und stellt ein Widerlager für den Kunststoff-Pufferkörper 5 dar, welcher auf Kompression gebracht wird. Bei Bewegung der Gesamtanordnung postoperativ dient sie als Gleitpartner zur Innenhülse, d.h. zum Hohlkugelsegment 3.

Das eigentliche Bewegungselement wird durch den Kunststoff-Pufferkörper 5 gebildet, und zwar bei Zug und Druck auf den Stößel bzw. den Verbinder 7.

Gemäß einem Ausführungsbeispiel der Erfindung ermöglicht die geschaffene Geometrie in Verbindung mit dem eingesetzten Material Polycarbonaturethan eine Verkürzung des Systems um ca. 2,2 mm und eine Verlängerung um ca. 2,8 mm.

Durch die Ausgestaltung des Stößels 6 mit Kopf 61 wird der Kunststoff-Pufferkörper 5 nur auf Druck belastet.

Der Verbinder 7 fungiert als Verbindungselement zur nächsten Pedikelschraube eines beliebigen Systems. Zum Zweck der Implantation ist der Stab mit allen daran verbundenen Komponenten nach oben in die Aussparung 8 verschwenkbar, um das bereits erwähnte Einschrauben der Pedikelschraube zu ermöglichen.

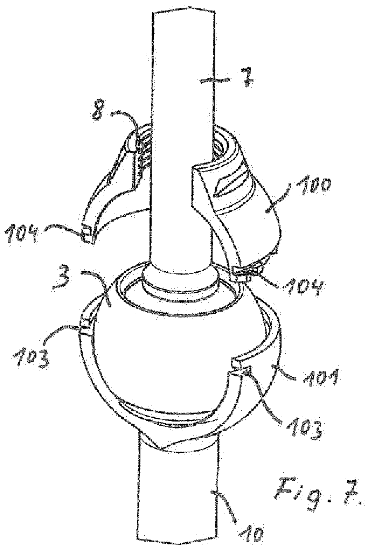

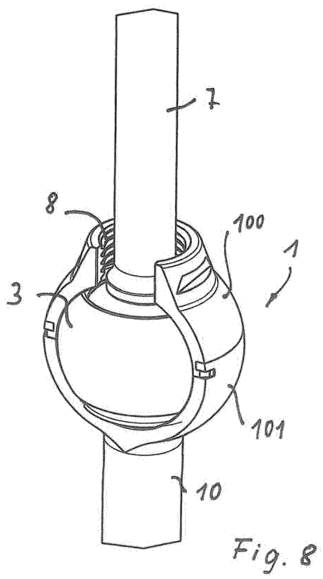

Die Fig. 7 und 8 zeigen eine Weiterbildung der Erfindung mit einer Pedikelschraube, die einen kugelsegmentförmigen Kopf 1 besitzt, welcher aus zwei Halbschalen bzw. Halbschalensegmenten 100; 101 besteht.

Die beiden Halbschalen 100; 101 weisen komplementäre Rastmittel 103 und Rastgegenmittel 104 auf, um selbige formschlüssig und/oder kraftschlüssig zu verbinden. Diese formschlüssige und/oder kraftschlüssige Verbindung geschieht bevorzugt unter Nutzung des Prinzips eines bekannten Bajonettverschlusses.

## Patentansprüche

1. Polyaxiale Pedikelschraube mit kugelsegmentförmigem Kopf (1) und Gewindeschraubenschaft (10), wobei im Kopf (1) ein Hohlkugelsegment (3) mit Ausnehmung zur Aufnahme eines stabförmigen Verbinders (7) gelagert ist, um Schwenk- und Drehbewegungen des Verbinders (7) zu ermöglichen, wobei weiterhin in Längsachsenrichtung des Gewindeschraubenschafts (10) der kugelsegmentförmige Kopf (1) eine Aussparung (8) mit Innengewinde aufweist, in welche der stabförmige Verbinder (7) hinein- und herausbewegbar ist und wobei mittels des Innengewindes in der Aussparung (8) eine Madenschraube oder dergleichen Mittel (9) zum Blockieren des Hohlkugelsegments (3) einbringbar ist,
**dadurch gekennzeichnet, dass**
im Hohlkugelsegment (3) ein Kunststoff-Pufferkörper (5) eingesetzt ist, welcher mit einem Stößel (6) in Verbindung steht, wobei der Stößel (6) einen Kopf (61) aufweist, welcher in eine diesbezügliche Ringnut im Kunststoff-Pufferkörper eingreift oder welcher vom Kunststoff-Pufferkörper mindestens teilweise umgeben ist, weiterhin der Kunststoff-Pufferkörper (5) einen umlaufenden Führungsring (51) und einen endseitigen sphärischen Führungsanschlag (52) besitzt, wobei dem Führungsanschlag (52) gegenüberliegend eine Öffnung für den Stößel (6) ausgebildet ist und sich der Rand (53) der Öffnung an einer Schale (4) abstützt, welche ihrerseits an der Innenseite des Hohlkugelsegments (3) anliegt, derart, dass der Kunststoff-Pufferkörper (5) mit seinem Führungsanschlag (52) im kugelsegmentförmigen Kopf (1) unter Vorspannung fixiert ist.

2. Polyaxiale Pedikelschraube nach Anspruch 1,
**dadurch gekennzeichnet, dass**
der Stößel (6) einen Abschnitt zum Befestigen des stabförmigen Verbinders (7) aufweist.

3. Polyaxiale Pedikelschraube nach Anspruch 1 oder 2,
**dadurch gekennzeichnet, dass**
im Führungsring (51) und/oder im Führungsanschlag (52) Nuten oder Aussparungen (54) zum Flüssigkeitsdurchtritt ausgebildet sind.

4. Polyaxiale Pedikelschraube nach einem der vorangegangenen Ansprüche,
**dadurch gekennzeichnet, dass**
über die Ausbildung des Kunststoff-Pufferkörpers (5) hinsichtlich Geometrie und/oder Material Radial-, Zug- und Druckkräfte und diesbezüglich erreichbare Hub- und Schwenkbewegungen des Stößels (6) vorgebbar sind.

5. Polyaxiale Pedikelschraube nach einem der vorangegangenen Ansprüche,
**dadurch gekennzeichnet, dass**
der Kunststoff-Pufferkörper (5) aus Polycarbonaturethan (PCU) besteht.

6. Polyaxiale Pedikelschraube nach einem der vorangegangenen Ansprüche,
**dadurch gekennzeichnet, dass**
das Hohlkugelsegment (3) mittels eines Klemmrings (2) im kugelsegmentförmigen Kopf (1) gesichert ist.

7. Polyaxiale Pedikelschraube nach einem der vorangegangenen Ansprüche,
**dadurch gekennzeichnet, dass**
das Hohlkugelsegment (3) aus Titanmaterial oder einer Titanlegierung besteht.

8. Polyaxiale Pedikelschraube nach einem der vorangegangenen Ansprüche,
**dadurch gekennzeichnet, dass**
die Schale (4) aus einer Titanlegierung oder einem Keramikmaterial besteht.

9. Polyaxiale Pedikelschraube nach einem der vorangegangenen Ansprüche,
**dadurch gekennzeichnet, dass**
der Gewindeschraubenschaft (10) ein selbstschneidendes Gewinde aufweist.

10. Polyaxiale Pedikelschraube nach einem der vorangegangenen Ansprüche,
**dadurch gekennzeichnet, dass**
der Führungsanschlag (52) einen Zugang zum Kopf (61) des Stößels (6) besitzt.

11. Polyaxiale Pedikelschraube nach einem der vorangegangenen Ansprüche,
**dadurch gekennzeichnet, dass**
der kugelsegmentförmige Kopf (1) geteilt, insbesondere zweigeteilt ausgebildet ist.

12. Polyaxiale Pedikelschraube nach Anspruch 11,
**dadurch gekennzeichnet, dass**
der kugelsegmentförmige Kopf (1) aus zwei Halbschalen (100; 101) besteht.

13. Polyaxiale Pedikelschraube nach Anspruch 12,
**dadurch gekennzeichnet, dass**
die Halbschalen (100; 101) formschlüssig über Rastmittel und Rastgegenmittel (103; 104) verbunden sind.

## Claims

1. A polyaxial pedicle screw with a head (1) in the shape of a ball segment and with a threaded screw shank (10), wherein a hollow ball segment (3) with a recess for receiving a rod-shaped connector (7) is mounted in the head, in order to permit pivoting movements and rotation movements of the connector (7), wherein moreover, in the direction of the longitudinal axis of the threaded screw shank (10), the head (1) in the shape of a ball segment has a cutout (8) with an inner thread in which the rod-shaped connector (7) can be moved in and out and, wherein by means of the inner thread in the cutout (8), a grub screw or similar means (9) can be introduced for blocking the hollow ball segment (3),
**characterized in that**
a plastic buffer body (5) is fitted in the hollow ball segment (3) and is connected to a ram (6), wherein the ram (6) has a head (61) which engages in an associated annular groove in the plastic buffer body or which is at least in part surrounded by the plastic buffer body, the plastic buffer body (5) moreover has a circumferential guide ring (51) and a spherical guide stop (52) at one end, wherein an opening for the ram (6) is formed lying opposite the guide stop (52), and the edge (53) of the opening bears on a shell (4), which in turn bears on the inner side of the hollow ball segment (3), in such a way that the plastic buffer body (5) with its guide stop (52) is fixed under pretension in the head (1) shaped as a ball segment.

2. The polyaxial pedicle screw according to claim 1,
**characterized in that**
the ram (6) has a section for fastening the rod-shaped connector (7).

3. The polyaxial pedicle screw according to claim 1 or 2,
**characterized in that**
grooves or cutouts (54) are formed in the guide ring (51) and/or guide slot (52) for fluid passage.

4. The polyaxial pedicle screw according to any one of the preceding claims,
**characterized in that**
via the design of the plastic buffer body (5) regarding geometry and/or material, radial, tensile and compressive forces and lifting and pivoting movements of the ram (6) may be predefined that can be achieved in this respect.

5. The polyaxial pedicle screw according to any one of the preceding claims,
**characterized in that**
the plastic buffer body (5) is made of polycarbonate urethane (PCU).

6. The polyaxial pedicle screw according to any one of the preceding claims,
**characterized in that**
the hollow ball segment (3) is secured within the head (1) shaped as a ball segment by means of a clamping ring (2).

7. The polyaxial pedicle screw according to any one of the preceding claims,
**characterized in that**
the hollow ball segment (3) is made of a titanium material or a titanium alloy.

8. The polyaxial pedicle screw according to any one of the preceding claims,
**characterized in that**
the shell (4) is made of a titanium alloy or a ceramic material.

9. The polyaxial pedicle screw according to any one of the preceding claims,
**characterized in that**
the threaded screw shank (10) has a self-tapping thread.

10. The polyaxial pedicle screw according to any one of the preceding claims,
**characterized in that**
the guide stop (52) has an access to the head (61) of the ram (6).

11. The polyaxial pedicle screw according to any one of the preceding claims,
**characterized in that**
the head (1) in the shape as a ball segment is formed to be divided, in particular divided in two.

12. The polyaxial pedicle screw according to claim 11,
**characterized in that**
the head (1) in the shape of a ball segment is composed of two half-shells (100; 101).

13. The polyaxial pedicle screw according to claim 12,
**characterized in that**
the half-shells (100; 101) are joined by locking means and counterlocking means (103; 104) in a form-fit manner.

## Revendications

1. Vis pédiculaire polyaxiale comportant une tête (1) en forme de segment de sphère et une tige filetée (10),
dans laquelle
un segment de sphère creuse (3) est monté dans la tête (1), qui est pourvu d'un évidement pour recevoir un connecteur (7) en forme de barreau pour permettre des mouvements de basculement et de rotation du connecteur (7),
en direction de l'axe longitudinal de la tige filetée (10), la tête (1) en forme de segment de sphère présente en outre une échancrure (8) pourvue d'un taraudage, dans laquelle le connecteur (7) en forme de barreau peut être inséré et extrait, et
à l'aide du taraudage dans l'échancrure (8), une vis sans tête ou un moyen similaire (9) peut être introduit(e) pour bloquer le segment de sphère creuse (3),
**caractérisée en ce que**
un corps tampon (5) en matière plastique est mis en place dans le segment de sphère creuse (3), qui est en liaison avec un poinçon (6),
le poinçon (6) présente une tête (61) qui s'engage dans une rainure annulaire correspondante dans le corps tampon en matière plastique ou qui est entourée au moins partiellement par le corps tampon en matière plastique,
le corps tampon (5) en matière plastique possède un anneau de guidage (51) périphérique et une butée de guidage (52) sphérique terminale,
une ouverture pour le poinçon (6) est ménagée à l'opposé de la butée de guidage (52), et le bord (53) de l'ouverture prend appui contre une coque (4) qui prend appui à son tour contre la face intérieure du segment de sphère creuse (3), de telle sorte que le corps tampon (5) en matière plastique est fixé sous précontrainte par sa butée de guidage (52) dans la tête (1) en forme de segment de sphère.

2. Vis pédiculaire polyaxiale selon la revendication 1,
**caractérisée en ce que**
le poinçon (6) présente une portion pour fixer le connecteur (7) en forme de barreau.

3. Vis pédiculaire polyaxiale selon la revendication 1 ou 2,
**caractérisée en ce que**
des rainures ou des échancrures (54) pour le passage de liquides sont ménagées dans l'anneau de guidage (51) et/ou dans la butée de guidage (52).

4. Vis pédiculaire polyaxiale selon l'une des revendications précédentes,
**caractérisée en ce que**
la réalisation du corps tampon (5) en matière plastique quant à la géométrie et/ou au matériau permet de déterminer des forces radiales, de traction et de compression et des mouvements de levage et de basculement du poinçon (6) qui peuvent ainsi être obtenus.

5. Vis pédiculaire polyaxiale selon l'une des revendications précédentes,
**caractérisée en ce que**
le corps tampon (5) en matière plastique est constitué en polycarbonate uréthane (PCU).

6. Vis pédiculaire polyaxiale selon l'une des revendications précédentes,
**caractérisée en ce que**
le segment de sphère creuse (3) est bloqué dans la tête (1) en forme de segment de sphère à l'aide d'un anneau de serrage (2).

7. Vis pédiculaire polyaxiale selon l'une des revendications précédentes,
**caractérisée en ce que**
le segment de sphère creuse (3) est constitué en matériau à base de titane ou en un alliage de titane.

8. Vis pédiculaire polyaxiale selon l'une des revendications précédentes,
**caractérisée en ce que**
la coque (4) est constituée d'un alliage de titane ou d'un matériau de céramique.

9. Vis pédiculaire polyaxiale selon l'une des revendications précédentes,
**caractérisée en ce que**
la tige filetée (10) comprend un filet autotaraudeur.

10. Vis pédiculaire polyaxiale selon l'une des revendications précédentes,
**caractérisée en ce que**
la butée de guidage (52) possède un accès à la tête (61) du poinçon (6).

11. Vis pédiculaire polyaxiale selon l'une des revendications précédentes,
**caractérisée en ce que**
la tête (1) en forme de segment de sphère est réalisée de façon divisée, en particulier de façon divisée en deux pièces.

12. Vis pédiculaire polyaxiale selon la revendication 11,
**caractérisée en ce que**
la tête (1) en forme de segment de sphère est constituée par deux demi-coques (100 ; 101).

13. Vis pédiculaire polyaxiale selon la revendication 12,
**caractérisée en ce que**
les demi-coques (100 ; 101) sont reliées en coopération de forme par des moyens d'enclenchement et par des moyens d'enclenchement antagonistes (103 ; 104).
